# EUROPEAN PATENT APPLICATION

(11) **EP 4 410 808 A1**
(43) Date of publication of application: **07.08.2024**
(21) Application number: 22876780.2
(22) Date of filing: 27.09.2022
(51) Int. Cl.: C07K 7/06, A61K 38/00, A61P 3/04, A61K 8/64, A61Q 19/00

(54) **PEPTIDE HAVING ANTI-OBESITY ACTIVITY AND USES THEREOF**

(30) Priority: 30.09.2021 KR 20210130419
(71) Applicant: Caregen Co., Ltd., Anyang-si, Gyeonggi-do 14119 (KR)
(72) Inventor: CHUNG, Yong Ji, Anyang-si, Gyeonggi-do 14119 (KR); KIM, Eun Mi, Anyang-si, Gyeonggi-do 14119 (KR); KIM, Seon Soo, Anyang-si, Gyeonggi-do 14119 (KR); JEONG, Ah Reum, Anyang-si, Gyeonggi-do 14119 (KR)
(74) Representative: Heinonen & Co
(86) International application number: PCT/KR2022/014393
(87) International publication number: WO 2023/055008

(57) **Abstract**

The present invention relates to a peptide having anti-obesity activity by reducing stored fat, and a composition for preventing, ameliorating, or treating obesity comprising same. Since the peptide comprising an amino acid sequence of SEQ ID NO: 1 according to the present invention has an activity to reduce fat stored in cells, the peptide may be used as a pharmaceutical for the purpose of preventing, ameliorating, or treating obesity or obesity-related diseases or as a raw material for cosmetics for the purpose of slimming down.

## Description

### TECHNICAL FIELD

The present invention relates to a peptide having anti-obesity activity and a composition for preventing, ameliorating, or treating obesity including the peptide as an active ingredient.

### BACKGROUND ART

Obesity refers to a state in which excess adipose tissue is present in the body as excess energy is stored as body fat when energy consumption from food is not balanced. According to the World Health Organization (WHO), more than 1 billion adults worldwide are overweight, of which at least 3 million are clinically obese, with a marked increase in the United States and Europe. Overweight and obesity increase blood pressure and cholesterol levels, causing various diseases such as heart disease, diabetes, and arthritis, and increasing the incidence of various adult diseases. In addition, overweight and obesity are one of the factors that increase the incidence of various adult diseases such as arteriosclerosis, hypertension, hyperlipidemia, or heart disease not only in adults but also in children and adolescents.

Representative anti-obesity drugs that are currently approved by the US FDA and widely prescribed include a group of drugs acting as appetite suppressants by acting on the central nervous system and orlistat (Xenical), which is an inhibitor of the digestive enzyme secreted from the pancreas (lipase). As for drugs that act on the central nervous system, the permission of a number of drugs such as sibutramine has been revoked due to their cardiovascular and psychiatric side effects. Orlistat has limitations in that it has various side effects and that its drug effect varies depending on the amount of fat intake. In contrast, liraglutide, which is a glucagon-like peptide-1 (GLP-1) receptor promoter, has been approved and used as an endocrine peptide target drug (Non-Patent Document 1), but the risk of thyroid cancer is emerging.

Accordingly, there is a demand for the development of a next-generation anti-obesity drug capable of overcoming the limitations of drugs acting on the central nervous system and gastrointestinal tract. Recently, the importance of development of anti-obesity drugs is increasing by showing a trend of switching the direction of development from the drugs acting on the central nervous system and gastrointestinal tract to those acting locally, and from small molecule synthetic compounds for oral administration to synthetic peptides for subcutaneous or intravascular injection. Additionally, development of new drugs, in which new biotechnologies such as therapeutic antibodies, RNAi, and aptamers are applied, is being sought. The development of synthetic peptide therapeutics is a promising driving force for the future biopharmaceutical industry, and the discovery of new drug targets and mechanisms, development of peptide-based drugs, and development of delivery optimization technology are very important due to the significant potential for clinical effectiveness in the field of development of obesity treatment.

### [Prior Art Document]

### [Non-Patent Document]

(Non-Patent Document 1) Pi-Sunyer X et al. A Randomized, Controlled Trial of 3.0 mg of Liraglutide in Weight Management. N Engl J Med. 2015; 373:11-22.

### DISCLOSURE OF THE INVENTION

### TECHNICAL PROBLEM

An object of the present invention is to provide a peptide having anti-obesity activity by promoting lipolysis.

Another object of the present invention is to provide a pharmaceutical composition for preventing or treating obesity, which includes the peptide having the above-described activity as an active ingredient.

Still another object of the present invention is to provide a composition for promoting lipolysis, which includes the peptide having the above-described activity as an active ingredient.

Still another object of the present invention is to provide a cosmetic composition for preventing or ameliorating obesity, which includes the peptide having the above-described activity as an active ingredient.

### TECHNICAL SOLUTION

In order to achieve the above objects, an aspect of the present invention provides a peptide including the amino acid sequence of SEQ ID NO: 1.

Another aspect of the present invention provides a pharmaceutical composition for preventing or treating obesity including the peptide as an active ingredient.

Still another aspect of the present invention provides a composition for promoting lipolysis including the peptide as an active ingredient.

Still another aspect of the present invention provides a cosmetic composition for preventing or ameliorating obesity including the peptide as an active ingredient.

### ADVANTAGEOUS EFFECTS

Since the peptide including the amino acid sequence of SEQ ID NO: 1 according to the present invention has the activity of reducing the fat accumulated in cells, the peptide may be used as a raw material for pharmaceutical drugs for the purpose of preventing, ameliorating, or treating obesity or obesity-related diseases or cosmetics for the purpose of body slimming.

The effects of the present invention are not limited to the effects mentioned above, and other effects not mentioned will be clearly understood by those skilled in the art from the following description.

### BRIEF DESCRIPTION OF THE DRAWINGS

FIG. 1A shows the results of RT-PCR confirming that the expression of ATGL, HSL, and perilipin1, which are genes related to the promotion of lipolysis, in adipocytes by treatment with the peptide of the present invention.
FIG. 1B shows the results confirming that the expression of ATGL, a gene related to the promotion of lipolysis, was increased in adipocytes by treatment with the peptide of the present invention.
FIG. 1C shows the results confirming that the expression of HSL, a gene related to the promotion of lipolysis, was increased in adipocytes by treatment with the peptide of the present invention.
FIG. 1D shows the results confirming that the expression of perilipin1, a gene related to the promotion of lipolysis, was increased in adipocytes by treatment with the peptide of the present invention.
FIG. 2A shows the results of Western blot confirming that the expression of pHSL, a protein related to the promotion of lipolysis, in adipocytes by the peptide of the present invention.
FIG. 2B shows the results of Western blot confirming that the expression of pHSL, a protein related to the promotion of lipolysis, was increased in adipocytes by the peptide of the present invention.
FIG. 3 shows the results of confirming the lipolytic effect of the peptide of the present invention by measuring the amount of glycerol liberated in adipocytes.
FIG. 4A shows the results of confirming the expression of genes related to thermogenesis in brown adipocytes by the peptide of the present invention in adipocytes by RT-PCR.
FIG. 4B shows the results of confirming the expression of PGC1α, a gene related to thermogenesis in brown adipocytes, was increased by the peptide of the present invention in adipocytes by RT-PCR.
FIG. 4C shows the results of confirming the expression of Tbx1, a gene related to thermogenesis in brown adipocytes, was increased by the peptide of the present invention in adipocytes by RT-PCR.
FIG. 4D shows the results of confirming the expression of FGF21, a gene related to thermogenesis in brown adipocytes, was increased by the peptide of the present invention in adipocytes by RT-PCR.
FIG. 4E shows the results of confirming the expression of UCP1, a gene related to thermogenesis in brown adipocytes, was increased by the peptide of the present invention in adipocytes by RT-PCR.
FIG. 5A shows the results of Western blot confirming the expression of proteins related to thermogenesis in brown adipocytes by the peptide of the present invention in adipocytes.
FIG. 5B shows the results of Western blot confirming the expression of PRDM16, a protein related to thermogenesis in brown adipocytes, was increased by the peptide of the present invention in adipocytes.
FIG. 5C shows the results of Western blot confirming the expression of UCP1, a protein related to thermogenesis in brown adipocytes, was increased by the peptide of the present invention in adipocytes.

### MODE FOR CARRYING OUT THE INVENTION

Hereinafter, the present invention will be described in detail.

An aspect of the present invention provides a peptide including the amino acid sequence represented by SEQ ID NO: 1.

As used herein, the term 'peptide' refers to a linear molecule consisting of amino acid residues, and specifically, the peptide of the present invention may include the amino acid sequence represented by SEQ ID NO: 1.

The 'peptide' may be amino acid variants or fragments having a different sequence by deletion, insertion, substitution, or a combination thereof within the range that does not affect the function. Amino acid exchanges that do not entirely alter the activity of the peptide are known in the art. In some cases, the peptide may be modified by phosphorylation, sulfation, acrylation, glycosylation, methylation, farnesylation, etc. Accordingly, the peptide of the present invention includes a peptide having an amino acid sequence substantially identical to that of the peptide including the amino acid sequence of SEQ ID NO: 1, and variants or active fragments thereof.

The substantially identical protein refers to an amino acid sequence having 75% or more, 80% or more, 85% or more, 90% or more, 95% or more, 98% or more, or 99% or more with the amino acid sequence of SEQ ID NO: 1, but is not limited thereto, and as long as the amino acid sequence has 75% or more amino acid sequence homology and has the same activity, it is included in the scope of the present invention.

In addition, the peptide of the present invention may further include a targeting sequence, a tag, a labeled residue, or an amino acid sequence prepared for a specific purpose to increase half-life or peptide stability.

In addition, in order to obtain better chemical stability, enhanced pharmacological properties (half-life, absorbency, potency, efficacy, *etc*.), altered specificity (*e*.*g*., broad biological activity spectrum), and reduced antigenicity, a protecting group may be bound to the N-terminus or C-terminus of the peptide according to the present invention. The 'stability' is used to mean not only *in vivo* stability that protects the peptide of the present invention from attack by proteases *in vivo,* but also storage stability (*e.g*., storage stability at room temperature).

The N-terminal modification may be one, in which a protecting group selected from the group consisting of an acetyl group, a fluorenylmethoxycarbonyl group, a formyl group, a palmitoyl group, a myristyl group, a stearyl group, and polyethylene glycol (PEG) is bound to the N-terminus of the peptide, but is not limited thereto.

The C-terminal modification may be one, in which a hydroxyl group (-OH), an amino group (-NH2), an azide (-NHNH₂), *etc.* is bound to the C-terminus of the peptide, but is not limited thereto.

Synthesis of the peptide of the present invention may be performed using, for example, a device or genetic engineering technique. In the case where the synthesis is performed using a device, a desired peptide may be synthesized using an Fmoc solid-phase method in an automatic peptide synthesizer.

In a specific embodiment of the present invention, the peptide including the amino acid sequence of SEQ ID NO: 1 of the present invention was synthesized and identified using the Fmoc solid-phase method, and selected by verifying the efficacy of the identified peptide.

In a specific embodiment of the present invention, peptides having anti-obesity activity were selected through validation of the lipolytic effect in order to verify the efficacy of the identified peptide.

In a specific embodiment of the present invention, it was confirmed that when adipocytes were treated with the peptide of the present invention, the accumulated fat was decomposed and the amount of glycerol released was increased.

In a specific embodiment of the present invention, it was confirmed that when adipocytes were treated with the peptide of the present invention, the expression of the lipolysis factor perilipin, hormone sensitive lipase (HSL), or adipose triglyceride lipase (ATGL) was increased.

In a specific embodiment of the present invention, it was confirmed that when adipocytes are treated with the peptide of the present invention, the expression of peroxisome proliferator-activated receptor gamma coactivator 1-alpha (PGC1α), T-box transcription factor (TBx1), fibroblast growth factor 21 (FGF21), uncoupling protein 1 (UCP1) or PRDI-BF1 and RIZ homology domain including 16 (PRDM16), which are factors related to the conversion of brown adipocytes, was increased.

These results indicate that the peptide of the present invention has an activity to reduce intracellular fat.

Another aspect of the present invention provides a pharmaceutical composition for preventing or treating obesity including a peptide, which includes the amino acid sequence of SEQ ID NO: 1, as an active ingredient.

As used herein, 'obesity' refers to a condition or disease in which body fat is excessively accumulated in the body due to energy imbalance, and specifically, 'obesity' may refer to deposition of fat, and furthermore, energy imbalance, and may be used as a meaning including 'obesity-related diseases' that may be caused by a condition with excessive body fat.

The 'deposition of fat' may refer to, for example, a condition selected from the group consisting of obesity, fat redistribution syndrome, lower eyelid fat herniation, lipoma, Dercum's disease, lipodystrophy, excessive visceral fat, macromastia, morbid obesity, diffuse body fat around the flanks and arms, and fat deposits related to cellulite.

The 'obesity-related diseases' may include hypertension, diabetes, an increased concentration of plasma insulin, insulin resistance, hyperlipidemia, metabolic syndrome, insulin resistance syndrome, obesity-related gastroesophageal reflux, arteriosclerosis, hypercholesterolemia, hyperuricemia, lower back pain, cardiac hypertrophy and left ventricular hypertrophy, lipodystrophy, non-alcoholic steatohepatitis, cardiovascular disease, polycystic ovary syndrome, etc.

As used herein, the term 'prevention' refers to any action that inhibits or delays the onset of obesity, and the term 'amelioration or treatment' includes all actions that beneficially change in relation to obesity, such as reduction, alleviation, or elimination of the symptoms of obesity, or delaying, stopping, or reversing the progression of obesity.

The peptide of the present invention not only significantly increases the expression of lipolysis promoting genes and proteins encoded by the genes, but also increases the expression of brown adipocyte conversion-related genes (thermogenesis-related genes) and proteins, and thus the peptide can control energy imbalance or allow to get out of a state with excessive body fat, thereby ameliorating or treating obesity.

In a specific embodiment of the present invention, the peptide can prevent, ameliorate, or treat obesity by increasing the expression of perilipin1, HSL, and/or ATGL in differentiation-induced pre-adipocytes.

In a specific embodiment of the present invention, the peptide can prevent, ameliorate, or treat obesity by increasing the expression of PGC1α, TBx1, FGF21, and UCP1 and/or PRDM.

The pharmaceutical composition for preventing or treating obesity of the present invention may further include a pharmaceutically acceptable carrier.

A pharmaceutically acceptable carrier may further include, for example, a carrier for oral administration or a carrier for parenteral administration. Carriers for oral administration may include lactose, starch, cellulose derivatives, magnesium stearate, stearic acid, etc. In addition, carriers for parenteral administration may include water, suitable oil, saline, aqueous glucose, glycol, etc. In addition, stabilizers and preservatives may be further included. Suitable stabilizers may include antioxidants such as sodium bisulfite, sodium sulfite, or ascorbic acid. Suitable preservatives may include benzalkonium chloride, methyl- or propyl-paraben, and chlorobutanol. Suitable pharmaceutically acceptable carriers may be referenced to those described in Remington's Pharmaceutical Sciences (19th ed., Mack Publishing Company, Easton, PA, 1995).

The pharmaceutical composition of the present invention may be administered to mammals including humans by any method. For example, the pharmaceutical composition may be administered orally or parenterally, and parenteral administration methods include, but are not limited to, intravenous, intramuscular, intraarterial, intramedullary, intrathecal, intracardiac, transdermal, subcutaneous, intraperitoneal, intranasal, enteral, topical, sublingual, or rectal administration.

The pharmaceutical composition of the present invention may be formulated into a preparation for oral administration or parenteral administration according to the administration route as described above, and the preparation for parenteral administration is specifically formulated in the form of an injection preparation or a preparation for external use.

When the pharmaceutical composition of the present invention is used as an injection preparation, it may be used for removing localized fat deposits.

The localized fat may be localized in a target area selected from the group consisting of the abdomen, an area under the eyes, an area under the chin, an area under the arms, buttocks, thighs, calves, back, and an area around the bra line.

The pharmaceutical composition of the present invention is administered in a pharmaceutically effective amount. In the present invention, 'pharmaceutically effective amount' means an amount sufficient to treat a disease with a reasonable benefit/risk ratio applicable to medical treatment, and the effective dose level may be determined according to factors including the type of the patient's disease, severity, activity of the drug, sensitivity to the drug, time of administration, route of administration and excretion rate, duration of treatment, drugs used concurrently, and other factors well known in the medical field. The pharmaceutical composition may be administered as an individual therapeutic agent or in combination with other anti-obesity therapeutic agents, may be administered simultaneously with, separately from, or sequentially with conventional therapeutic agents, and may be administered once or multiple times. Considering all of the above factors, it is important to administer an amount that can obtain the maximum effect with the minimum amount without side effects, which can easily be determined by those skilled in the art.

The effective amount of the pharmaceutical composition may vary depending on the patient's age, sex, condition, body weight, absorption rate of active ingredients in the body, inactivity rate, excretion rate, disease type, drugs used in combination, and may increase or decrease depending on the route of administration, severity of obesity, sex, weight, age, etc., and may be administered, for example, about 0.0001 µg to about 500 mg, preferably 0.01 µg to 100 mg per 1 kg of the patient's body weight per day.

Still another aspect of the present invention provides a composition for promoting lipolysis including a peptide, which includes the amino acid sequence represented by SEQ ID NO: 1 as an active ingredient.

The composition may be for removing localized fat deposits.

The localized fat may mean being localized in a target region selected from the group consisting of the abdomen, an area under the eyes, an area under the chin, an area under the arms, buttocks, thighs, calves, back, and an area around the bra line.

Still another aspect of the present invention provides a cosmetic composition for preventing or ameliorating obesity including a peptide, which includes the amino acid sequence of SEQ ID NO: 1 as an active ingredient.

The cosmetic composition may be prepared in any formulation commonly prepared in the art, for example, a solution, a suspension, an emulsion, a paste, a gel, a cream, a lotion, a powder, a soap, a surfactant-including cleansing, an oil, a powder foundation, an emulsion foundation, a wax foundation, a spray, *etc.*, but is not limited thereto.

The cosmetic composition may be prepared in various forms such as a solution, a sol gel, an emulsion, an oils, a wax, and an aerosol, such as a softening lotion, a nutrient lotion, a nutrient cream, a massage cream, an essence, an eye cream, a cleansing cream, a cleansing foam, cleansing water, a mask, a spray, a powder, a hair tonic, a hair cream, a hair lotion, a hair shampoo, a hair conditioner, a hair spray, a hair aerosol, a pomade, and a gel, but is not limited thereto.

The cosmetic composition of the present invention may include other additives such as excipients, carriers, etc., and it is possible to apply and blend ordinary ingredients formulated in general skin cosmetics as needed.

When the formulation of the cosmetic composition is a paste, cream, or gel, it is possible to use an animal oil, a vegetable oil, a wax, paraffin, starch, tragacanth, a cellulose derivative, polyethylene glycol, silicone, bentonite, silica, talc, zinc oxide, etc. as a carrier component.

When the form of the formulation of the cosmetic composition is a powder or spray, it is possible to use lactose, talc, silica, aluminum hydroxide, calcium silicate, or polyamide powder as a carrier component, and particularly in the case of a spray, a propellant such as chlorofluorohydrocarbon, propane/butane, or dimethyl ether may be further included, but is not limited thereto.

When the form of the formulation of the cosmetic composition is a solution or emulsion, it is possible to use a solvent, a solubilizing agent, or an emulsifying agent as a carrier component, for example, water, ethanol, isopropanol, ethyl carbonate, ethyl acetate, benzyl alcohol, benzyl benzoate, propylene glycol, 1,3-butylglycol oil, glycerol aliphatic ester, fatty acid ester of polyethylene glycol or sorbitan, *etc.* may be used.

When the form of the formulation of the cosmetic composition is a suspension, it is possible to use, as a carrier component, a liquid diluent (*e.g.*, water, ethanol, or propylene glycol), a suspending agent (*e.g*., an ethoxylated isostearyl alcohol, polyoxyethyl sorbitol ester, polyoxyethylene sorbitan ester, etc.), microcrystalline cellulose, aluminum metahydroxide, bentonite, agar, tragacanth, *etc*.

When the formulation of the cosmetic composition is a surfactant-including cleansing, it is possible to use, as a carrier component, aliphatic alcohol sulfate, aliphatic alcohol ether sulfate, sulfosuccinic acid monoester, isethionate, an imidazolinium derivative, methyl taurate, sarcosinate, fatty acid amide ether sulfate, alkylamidobetaine, aliphatic alcohol, fatty acid glyceride, fatty acid diethanolamide, a vegetable oil, a lanolin derivative, an ethoxylated glycerol fatty acid ester, etc.

When the formulation of the cosmetic composition is a hair shampoo, base components for forming the shampoo (e.g., a thickener, a surfactant, a viscosity modifier, a moisturizer, a pH modifier, a preservative, an essential oil, etc.) may be mixed with the peptide of the present invention. CDE may be used as the thickener; LES, which is an anionic surfactant, and coco betaine, an amphoteric surfactant, may be used as the surfactant, and polyquater may be used as the viscosity modifier; glycerin may be used as the moisturizer; and citric acid and sodium hydroxide may be used as the pH modifier. A grapefruit extract, etc. may be used as the preservative, and in addition, essential oils (*e.g.*, cedarwood, peppermint, rosemary, *etc*.), silk amino acids, pentaol, or vitamin E may be added.

The components to be included in the cosmetic composition are active ingredients, and the cosmetic composition may further include, in addition to the peptide of the present invention and carrier components, conventional adjuvants commonly used in cosmetic compositions (e.g., antioxidants, stabilizers, solubilizers, vitamins, pigments, and fragrances), but are not limited thereto.

As described above, since the peptide of the present invention has the effect of promoting the decomposition of the fat accumulated in cells, it may be used as a raw material for pharmaceuticals for the purpose of preventing, ameliorating, or treating obesity or obesity-related diseases or cosmetics for the purpose of body slimming.

Still another aspect of the present invention provides a method for preventing or treating obesity, which includes administering, to a subject, a therapeutically effective amount of the peptide including the amino acid sequence of SEQ ID NO: 1.

Still another aspect of the present invention provides a method for promoting lipolysis, which includes administering, to a subject, a therapeutically effective amount of the peptide including the amino acid sequence of SEQ ID NO: 1.

Since the peptide including the amino acid sequence of SEQ ID NO: 1 is the same as the peptide described in the section of the "pharmaceutical composition for preventing or treating obesity" above, the details described above are used for specific description.

As used herein, the term "therapeutically effective amount" refers to the amount of a peptide that achieves the goal of ameliorating the incidence of a disease during treatment with the peptide, but avoids the harmful side effects usually associated with other treatments.

Still another aspect of the present invention provides a use of the amino acid sequence of SEQ ID NO: 1 for use in the preparation of a medicament for the prevention or treatment of obesity.

Hereinafter, the present invention will be described in detail by Examples and Experimental Examples.

However, the following Examples and Experimental Examples are only for exemplifying the present invention, and the content of the present invention is not limited by the following Examples and Experimental Examples.

### MODE FOR CARRYING OUT THE INVENTION

### [Synthesis Example 1]

### Synthesis of Peptides Including Amino Acid Sequence of SEQ ID NO: 1

A peptide having the amino acid sequence of SEQ ID NO: 1 shown in Table 1 below was synthesized using an automatic peptide synthesizer (Liberty, CEM Corporation, USA), and the synthesized peptide was purified using C18 reverse phase high performance liquid chromatography (HPLC) (U-3000, Thermo Fisher Scientific, USA). The column used was Pursuit XRs C18 (250 mm × 4.65 mm 100Å, Agilent, USA).

**[Table 1]**

| SEQ ID NO | Amino Acid Sequence of Peptide |
|---|---|
| 1 | DYNMKWIHVY |

### [Preparation Example 1]

### Preparation of pre-adipocytes and induction of differentiation into adipocytes

First, in order to differentiate pre-adipocytes into adipocytes, pre-adipocytes and 3T3-L1 cells purchased from the American Type Culture Collection (ATCC) were cultured in DMEM including 2% fetal bovine serum (FBS). In order to differentiate 3T3-L1 cells into adipocytes, the culture was dispensed into a 12-well plate at a density of 1 × 10⁵ cells/well, and 2 days after the time-point of achieving the confluent growth (day 0), the culture medium was replaced with DMEM medium including 10% FBS, 10 µg/mL insulin, 0.5 mM isobutylmethylxanthine (IBMX)(Sigma, USA), and 1 µM dexamethasone (Sigma, USA), and cultured. After 2 days (day 2), the culture medium was replaced with DMEM medium including 10% FBS and 10 ug/mL insulin and cultured, and 3 days thereafter (day 5), the culture medium was replaced with a medium including only 10% FBS. Two days thereafter (day 7), the cells were named 3T3-L1 adipocytes and cultured under the conditions of 37°C, 5% CO₂ environment. The adipocytes which were induced to differentiate for 10 days were used in subsequent experiments.

### [Experimental Example 1]

### Confirmation of changes in expression of lipolysis-related genes in adipocytes

In adipocytes, in order to confirm whether the peptide of the present invention has the effect of activating the lipolysis-promoting cell signaling pathway, mRNA expression levels of HSL, ATGL, and perilipin1 genes associated with lipolysis promotion were examined.

Specifically, adipocytes differentiated through Preparation Example 1 were treated with 5 µM and 50 µM of the peptide of SEQ ID NO: 1 and 20 nM of TNF-α, as a positive control, cultured for 72 hours, and then the cells were recovered and total RNA was extracted with Trizol (Thermo Fisher Scientific, USA). DNA complementary to the extracted RNA was obtained using TOPScript^{™} RT DryMIX (enzynomics, Korea), and the DNA was subjected to polymerase chain reaction (PCR) using TOPsimple^{™} DryMIX-nTaq (enzynomics, Korea), and thereafter, the PCR products were loaded on a 1.5% agarose gel to compare the mRNA expression levels of genes associated with the promotion of lipolysis. GAPDH gene was used as an internal control, and the primers used in the experiment are listed in [Table 2].

**[Table 2]**

| **Genes** | **Primers** | **Sequence (5'->3')** | **SEQ ID NO** |
|---|---|---|---|
| Perilipin1 | F | AAGGATCCTGCACCTCACAC | 2 |
| | R | CCTCTGCTGAAGGGTTATCG | 3 |
| HSL | F | GGACACACACACACCTG | 4 |
| | R | CCCTTTCGCAGCAACTTTAG | 5 |
| ATGL | F | TCGTGGATGTTGGTGGAGCT | 6 |
| | R | TGTGGCCTCATTCCTCCTA | 7 |
| GAPDH | F | GTGATGGCATGGACTGTGGT | 8 |
| | R | GGAGCCAAAAGGGTCATCAT | 9 |

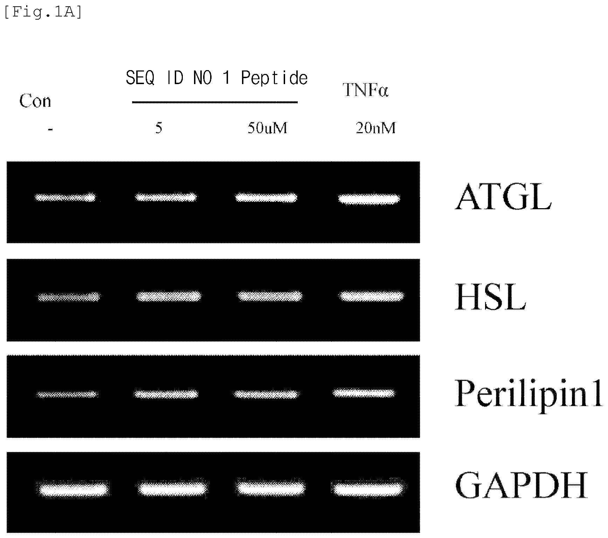

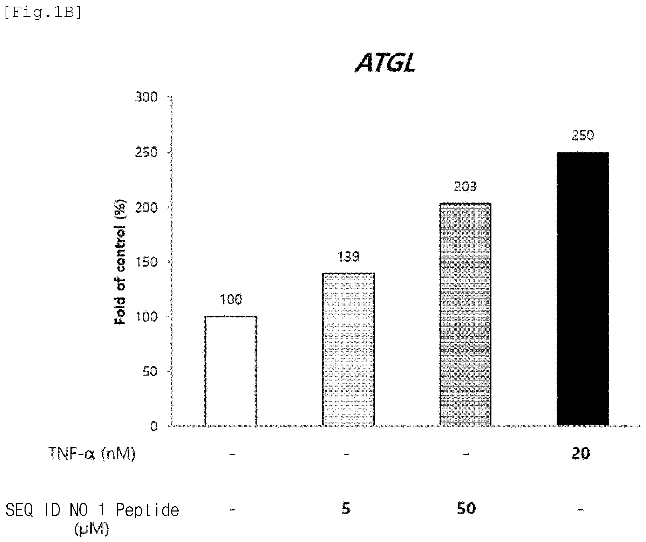

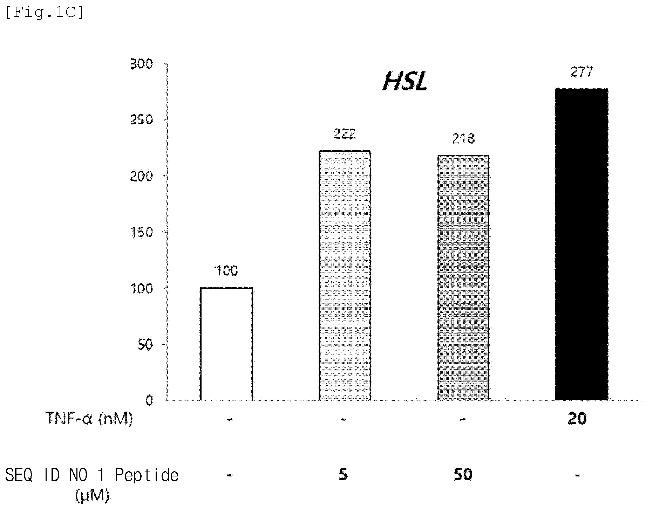

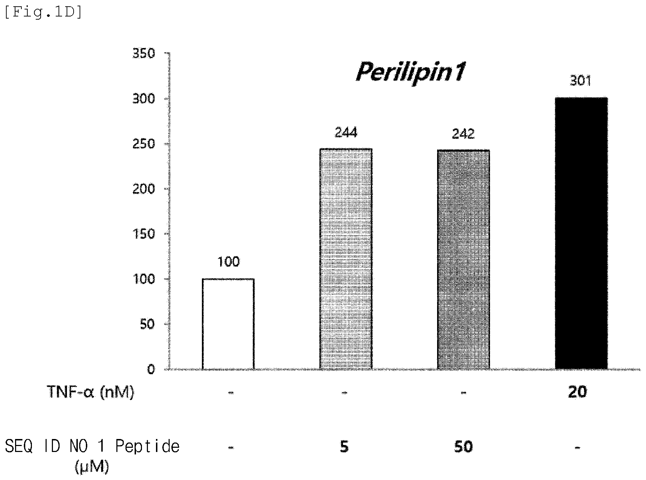

As a result, as shown in FIG. 1A to FIG. 1D, the expression of HSL, ATGL, and perilipin1 genes associated with the promotion of lipolysis was increased after treatment with the peptide. From this, it can be seen that the peptide of the present invention can increase the expression of the genes associated with the promotion of lipolysis so as to very effectively decompose fat.

### [Experimental Example 2]

### Confirmation of changes in expression of lipolysis-related proteins in adipocytes

In adipocytes, in order to confirm whether or not the peptide of the present invention has the effect of activating the lipolysis-promoting cell signaling pathway, the expression level of HSL, which is a protein associated with the promotion of lipolysis, was confirmed.

Specifically, the adipocytes differentiated in Preparation Example 1 were treated with 5 µM and 50 µM of the peptide of SEQ ID NO: 1, and after 1 hour, the cells were recovered and treated with a cell lysis buffer to obtain a lysate. Then, the protein was quantified and subjected to Western blotting using an anti-pHSL antibody (Cell Signaling Technology (CST), USA).

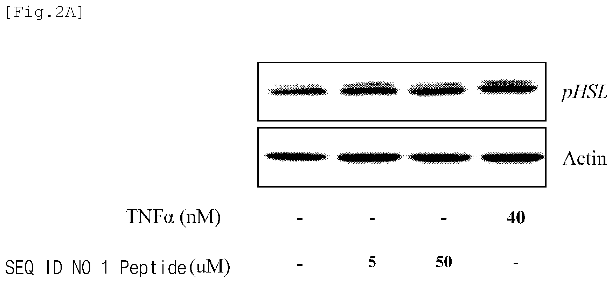

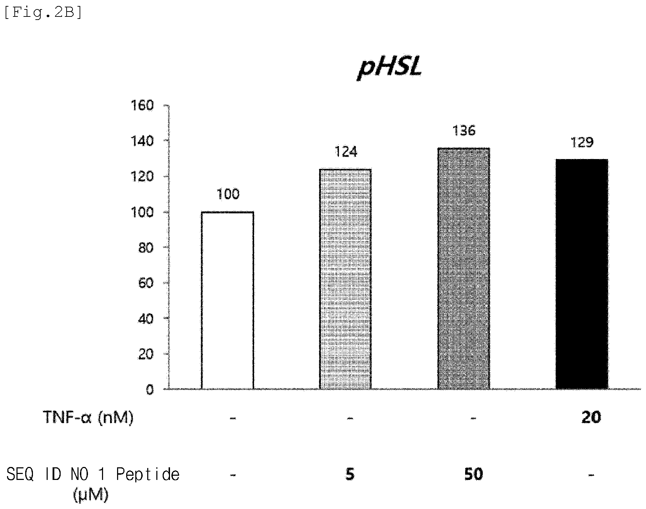

As a result, as shown in FIGS. 2A and 2B, phosphorylation of HSL protein associated with promotion of lipolysis was increased after treatment with the peptide.

Through this, it can be seen that the peptide of the present invention increases the expression of proteins associated with the promotion of lipolysis so that fat can be decomposed very effectively.

### [Experimental Example 3]

### Measurement of amount of glycerol, which is a lipolysis product, in adipocytes

Neutral fat accumulated in adipocytes is broken down into fatty acids and glycerol for energy production, cell signal transmission, *etc.* Therefore, the effect of decomposing neutral fat accumulated in adipocytes was confirmed by measuring the amount of free glycerol released.

Specifically, adipocytes differentiated in Preparation Example 1 were treated with the peptide of SEQ ID NO: 1 at concentrations of 5 µM and 50 µM, and treated with 20 nM of TNFα, as a positive control, followed by culturing for 48 hours. After obtaining the supernatant, the amount of glycerol was measured using a glycerol colorimetric assay kit (Cayman Chemical, USA).

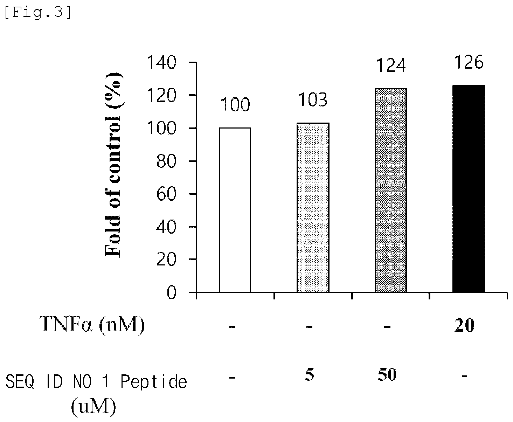

As a result, as shown in FIG. 3, it was confirmed that when treated with the peptide of SEQ ID NO: 1, the amount of glycerol released as fat was decomposed increased in a concentration-dependent manner of the peptide.

Through this, it can be seen that the peptide of the present invention has the activity to decompose neutral fat accumulated in adipocytes.

### [Experimental Example 4]

### Confirmation of increased expression of thermogenesis-related genes by the peptide of the present invention

In order to confirm whether the peptide of the present invention has the effect of preventing or treating obesity, the levels of mRNA expression of PGClα, Tbx1, FGF21, and UCP1, which are thermogenesis-related genes in brown adipocytes, were examined.

Specifically, the adipocytes obtained in Preparation Example 1 were treated with 5 µM and 50 µM of the peptide of SEQ ID NO: 1 and 20 nM of CL 316,243 (Sigma, C5976), as a positive control, cultured for 72 hours, and then the cells were recovered and total RNA was extracted with Trizol (Thermo Fisher Scientific, USA). DNA complementary to the extracted RNA was obtained using TOPScript^{™} RT DryMIX (enzynomics, Korea), and the DNA was subjected to polymerase chain reaction (PCR) using TOPsimple^{™} DryMIX-nTaq (enzynomics, Korea), and thereafter, the PCR products were loaded on a 1.5% agarose gel to compare the mRNA expression levels of thermogenesis-related genes in brown adipocytes. GAPDH gene was used as an internal control, and the primers used in the experiment are listed in [Table 3].

**[Table 3]**

| **Gene** | **Primer** | **Sequence (5'->3')** | **SEQ ID NO** |
|---|---|---|---|
| *PGClα* | F | ATGTGCAGCCAAGACTCTGTA | 10 |
| | R | CGCTACACCACTTCAATCCAC | 11 |
| *Tbx1l* | F | CTGTGGGACGAGTTCAATCAG | 12 |
| | R | TTGTCATCTACGGGCACAAAG | 13 |
| *FGF21* | F | CGTCTGCCTCAGAAGGACTC | 14 |
| | R | TCTACCATGCTCAGGGGGTC | 15 |
| *UCP1* | F | CCTGCCTCTCTCGGAAACAA | 16 |
| | R | GTAGCGGGGTTTGATCCCAT | 17 |

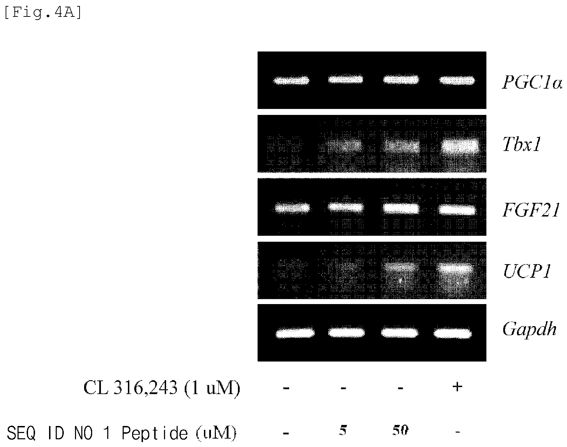

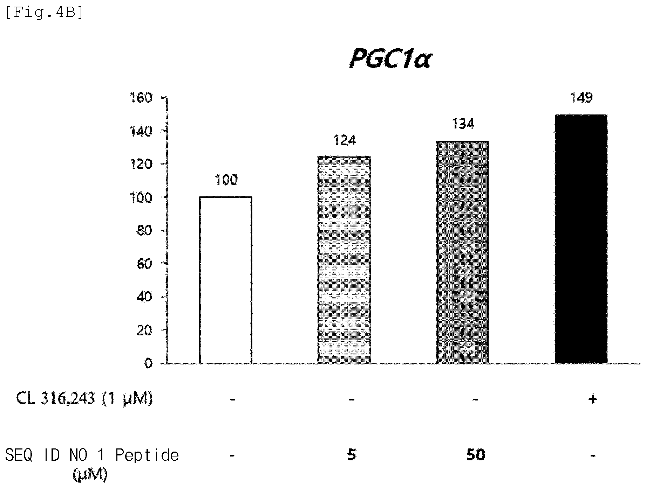

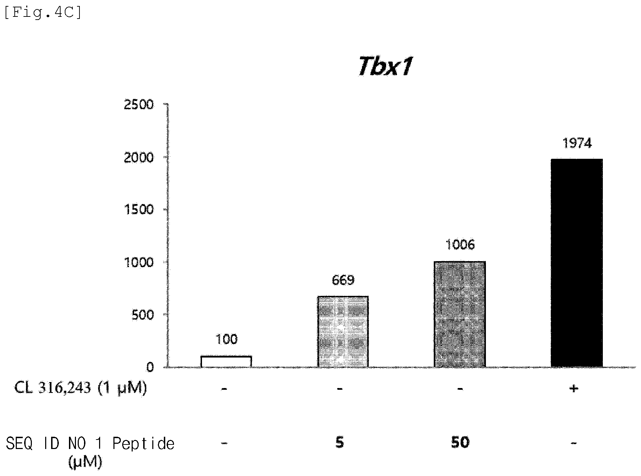

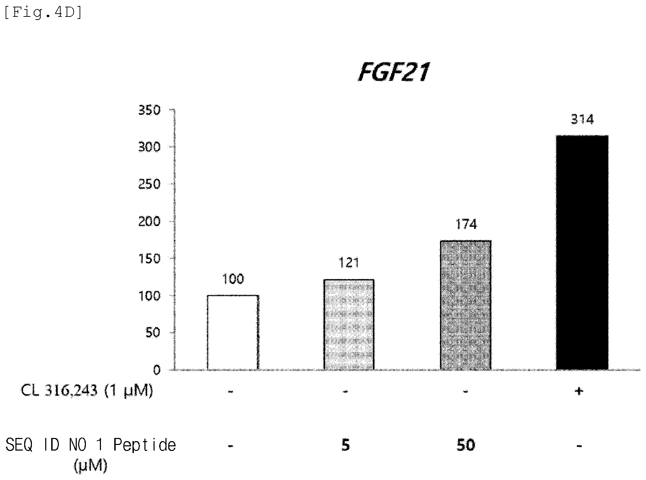

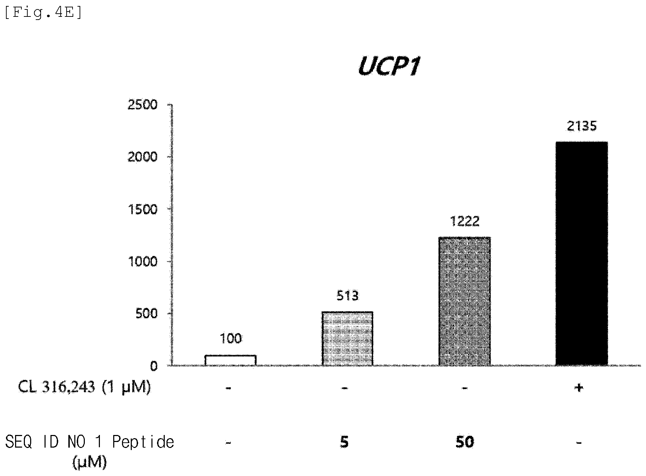

As a result, as shown in FIGS. 4A to 4E, it was confirmed that the expression of PGClα, Tbx1, FGF21, and UCP1 genes associated with thermogenesis in brown adipocytes was increased after treatment with the peptide of SEQ ID NO: 1. Through this, it can be seen that the peptide of the present invention is effective in preventing or treating obesity by increasing the expression of thermogenesis-related genes thereby increasing beta oxidation of free fatty acids.

### [Experimental Example 5]

### Confirmation of increased expression of thermogenesis-related proteins by the peptide of the present invention

In order to confirm whether the peptides of the present invention have an effect of preventing or treating obesity, the expression levels of PRDM16 and UCP1, which are thermogenesis-related proteins in brown adipocytes, were examined.

Specifically, the adipocytes obtained in Preparation Example 1 were treated with 5 µM and 50 µM of the peptide of SEQ ID NO: 1, respectively. After 24 hours, the cells were collected and treated with cell lysis buffer to obtain a lysate. Then, the protein was quantified and subjected to Western blotting was performed using an anti-PRDM16 antibody (Cell signaling technology (CST), USA) and an anti-UCP1 antibody (Abcam, England).

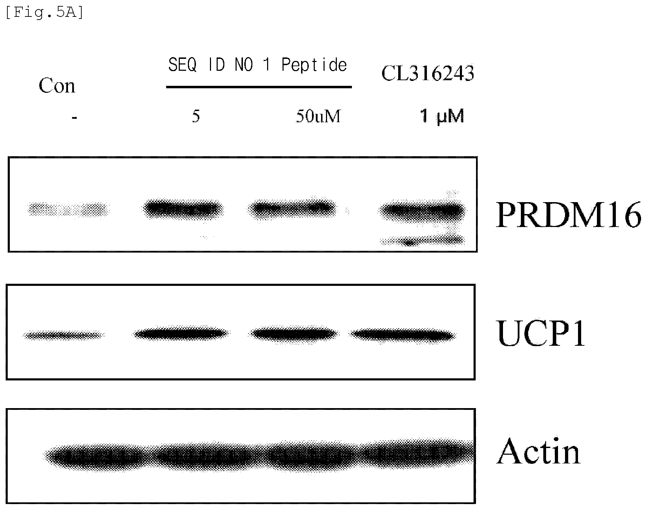

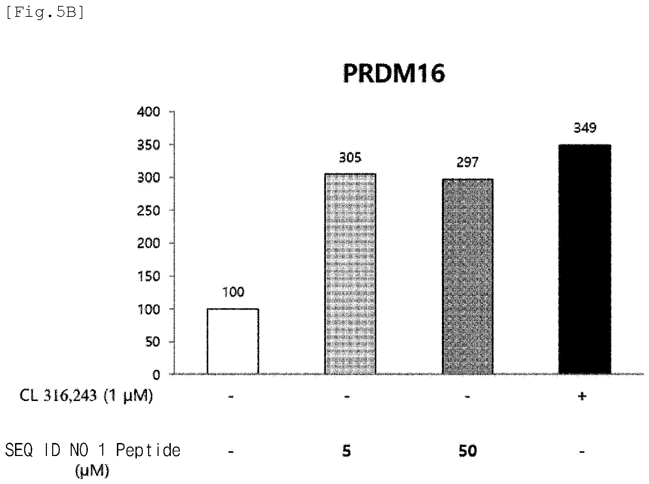

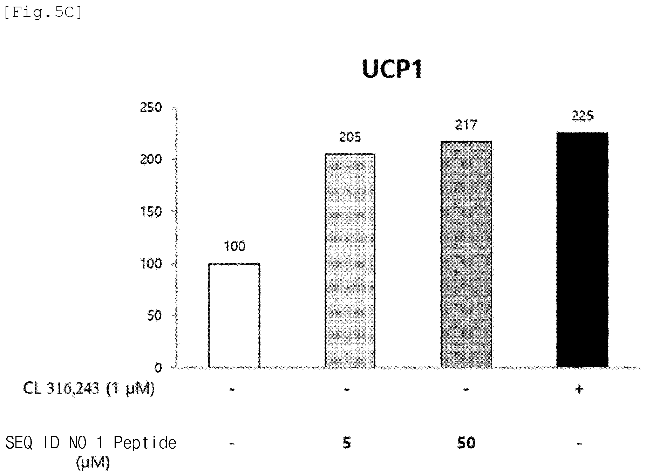

As a result, as shown in FIGS. 5A to 5C, the expression of a thermogenic marker protein that increases beta oxidation of free fatty acids was increased after treatment with the peptide.

Through this, it can be seen that the peptide of the present invention increases the expression of proteins associated with fat oxidation so that fat can be decomposed very effectively.

From the above, the present invention has been described in detail only in relation to the described embodiments, but it is apparent to those skilled in the art that various changes and modifications are possible within the scope of the technical spirit of the present invention, and it is natural that these changes and modifications fall within the scope of the appended claims.

## Claims

1. A peptide comprising the amino acid sequence represented by SEQ ID NO: 1.

2. The peptide of claim 1, wherein the peptide has the activity of reducing the fat accumulated in cells.

3. A pharmaceutical composition for preventing or treating obesity comprising the peptide of claim 1 as an active ingredient.

4. The pharmaceutical composition of claim 3, wherein the composition increases the expression of perilipin1, hormone sensitive lipase (HSL), or adipose triglyceride lipase (ATGL), which are factors associated with lipolysis.

5. The pharmaceutical composition of claim 3, wherein the composition increases the expression of peroxisome proliferator-activated receptor gamma coactivator 1-alpha (PGC1α), T-box transcription factor (TBx1), fibroblast growth factor 21 (FGF21), uncoupling protein 1 (UCP1), or PRDI-BF1 and RIZ homology domain containing 16 (PRDM16), which are factors associated with thermogenesis.

6. The pharmaceutical composition of claim 3, wherein the pharmaceutical composition is formulated in the form of a preparation for oral administration, a preparation for injection, or a preparation for external use.

7. A composition for promoting lipolysis comprising the peptide of claim 1 as an active ingredient.

8. The composition of claim 7, wherein the composition is for removing localized fat deposits.

9. The of claim 8, wherein the localized fat is localized in a target area selected from the group consisting of the abdomen, an area under the eyes, an area under the chin, an area under the arms, buttocks, thighs, calves, back, and an area around the bra line.

10. A cosmetic composition for preventing or ameliorating obesity comprising the peptide of claim 1 as an active ingredient.

11. The cosmetic composition of claim 10, wherein the cosmetic composition has one or more formulations selected from the group consisting of toners, lotions, creams, essences, emulsions, gels, hand creams, lipsticks, cleansing foams, cleansing creams, cleansing water, sprays, shampoos, conditioners, treatments, body cleansers, soaps, masks, massage agents, face powders, compacts, foundations, two-way cakes, and make-up bases.

The present invention relates to a peptide having anti-obesity activity by reducing stored fat, and a composition for preventing, ameliorating, or treating obesity comprising same. Since the peptide comprising an amino acid sequence of SEQ ID NO: 1 according to the present invention has an activity to reduce fat stored in cells, the peptide may be used as a pharmaceutical for the purpose of preventing, ameliorating, or treating obesity or obesity-related diseases or as a raw material for cosmetics for the purpose of slimming down.
